Europäisches Patentamt

European Patent Office

Office européen des brevets

(11) Veröffentlichungsnummer: **0 217 317 B1**

# (12) EUROPÄISCHE PATENTSCHRIFT

(45) Veröffentlichungstag der Patentschrift: **17.06.92**

(21) Anmeldenummer: **86113302.3**

(22) Anmeldetag: **26.09.86**

(51) Int. Cl.5: **A61B 17/58**

(54) **Schenkelhalsimplantat.**

(30) Priorität: **28.09.85 DE 3534747**

(43) Veröffentlichungstag der Anmeldung:
**08.04.87 Patentblatt 87/15**

(45) Bekanntmachung des Hinweises auf die
Patenterteilung:
**17.06.92 Patentblatt 92/25**

(84) Benannte Vertragsstaaten:
**AT BE CH DE FR GB IT LI LU NL SE**

(56) Entgegenhaltungen:
**AT-B- 236 030         DE-A- 2 051 289**
**DE-C- 931 431         DE-C- 1 046 827**
**DE-U- 8 213 228       FR-A- 2 501 032**

(73) Patentinhaber: **Von Hasselbach, Christoph, Dr.
med.
Armstrasse 41
WE-4300 Essen-Borbeck(DE)**

(72) Erfinder: **Von Hasselbach, Christoph, Dr.
med.
Armstrasse 41
WE-4300 Essen-Borbeck(DE)**

(74) Vertreter: **Ostriga, Harald, Dipl.-Ing. et al
Patentanwälte Dipl.-Ing. Harald Ostriga
Dipl.-Ing. Bernd Sonnet Stresemannstrasse
6-8
W-5600 Wuppertal 2(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft ein Schenkelhalsimplantat, wie es im Oberbegriff des Patentanspruchs 1 beschrieben und durch offenkundige Vorbenutzung bekanntgeworden ist.

Die Schenkelhalsplatte des vorbekannten Schenkelhalsimplantats weist zumeist einen U-förmigen Querschnitt auf. Die Stegaußenseite des U-Profils bildet die außenseitige Anlagefläche am Femur. Einstückig mit dem oberen Ende der Schenkelhalsplatte verbunden ist der Schaft, welcher beim bekannten Schenkelhalsimplantat mit Klinge bezeichnet wird. Die Klinge weist einen U-bis I-förmigen Querschnitt auf. Ein der offenkundigen Vorbenutzung weitgehend entsprechendes Schenkelhalsimplantat ist auch in der DE-A-2 051 289 (Seite 1, Abs. 2) erwähnt.

Bei dem bekannten Schenkelhalsimplantat verläuft der Implantationsvorgang wie folgt: bei einem pertrochantären oder bei einem Schenkelhalsbruch wird mit einem der Querschnittsform der Klinge angepaßten Stemmwerkzeug ein Aufnahmekanal subtrochantär und mit erforderlicher Axiallänge - zum Beispiel nur bis in den Schenkelhals oder gegebenenfalls bis in den Femurkopf hinein - von Hand eingebracht. Daraufhin wir die Klinge in diesen knöchernen Aufnahmekanal eingeschoben, worauf die Schenkelhalsplatte mit Kortikalisschrauben außen am Femur befestigt wird.

Das bekannte Implantat wird zunächst als unzulänglich empfunden, weil die vorbeschriebene Herstellung des Aufnahmekanals für die Klinge sehr arbeits- und zeitaufwendig ist. Außerdem können sich bei der Herstellung des Kanals sehr leicht Winkelfehlstellungen, Achsenversetzungen sowie zusätzliche Knochenabsplitterungen ergeben.

Der hauptsächliche Nachteil des bekannten Schenkelhalsimplantats besteht jedoch darin, daß dessen Anwendung bei instabilen pertrochantären Frakturen, etwa des Typs Evans III und IV, mit großem operativen Aufwand zwar möglich, Jedoch eine sofortige bzw. primäre postoperative Belastung unmöglich ist bei Einhaltung des physiologischen Schenkelhalswinkels. Eine primäre postoperative Belastung würde vielmehr einen mechanischen Zusammenbruch der zuvor vorgenommenen Osteosynthese zur Folge haben. Da aber vornehmlich ältere Menschen instabile pertrochantäre Frakturen erleiden, bei Anwendung des vorbekannten Implantats etliche Tage nach der Operation aber nicht aufstehen dürfen, besteht bei diesen Patienten eine erhöhte Gefahr von durch Immobilität hervorgerufenen Komplikationen, beispielsweise in Form einer Lungenentzündung.

Ausgehend von dem eingangs beschriebenen vorbekannten Schenkelhalsimplantat, liegt daher der Erfindung die Aufgabe zugrunde, ein mit geringem operativen Aufwand einsetzbares Schenkelhalsimplantat zu schaffen, welches unter Einhaltung des physiologischen Schenkelhalswinkels auch bei instabilen pertrochantären Frakturen eine primäre postoperative Belastung gestattet, ohne daß die Gefahr eines mechanischen Zusammenbruchs der vorgenommenen Osteosynthese besteht. Diese Aufgabe wurde entsprechend dem Kennzeichenteil des Hauptanspruchs gelöst.

Die Eigenart des erfindungsgemäßen Schenkelhalsimplantats kann wie folgt erklärt werden:

Da der subtrochantär zu implantierende Schaft durchgehend glatt und kreiszylindrisch ausgebildet ist, genügt als Aufnahmekanal innerhalb des Knochens eine leicht mit einem Knochenbohrer einzubringende Bohrung mit kreisförmigem Querschnitt. Hierbei wird nur soviel Knochensubstanz wie unbedingt nötig entfernt. Es ist nur darauf zu achten, daß wegen etwaiger Setzungen des Kopf-Hals-Fragments die Axiallänge des Bohr- bzw. Aufnahmekanals die glatte Schaftlänge um ca. 1 cm übersteigt. Der Einsatz eines mechanisch angetriebenen Knochenbohrers zur Schaffung der Schaft-Aufnahmebohrung hat zugleich den Vorteil, daß während des Einbringens der Bohrung mit sehr geringer axialer Vorschubkraft gearbeitet und so eine Verschiebung einzelner Knochenfragmente bei instabilen Frakturen vermieden werden kann. Die oberhalb des Schaftes pertrochantär verlaufenden Spongiosa-Schrauben sind über den einstückig und biegesteif an den oberen Bereich der Schenkelhalsplatte angeschlossenen Fortsatz in den Kraftfluß des eine insgesamt zusammenhängende statische Einheit darstellenden Schenkelhalsimplantats integriert. Die Spongiosa-Schrauben dienen einerseits dazu, die unter Gleitpassung auf dem Schaft sitzenden oder doch mittelbar vom Schaft geführten Knochenfragmente aneinanderzuziehen bzw. zu komprimieren und andererseits den Schaft durch Aufnahme von Zugkräften von einer schädlichen Biegespannung zu entlasten. Der erfindungsgemäße Schaft stellt eine Überbrückungsosteosynthese her. Und zwar findet eine Überbrückung der Knochenfragmente zwischen dem fest mit der Schenkelhalsplatte verschraubten schaftseitigen Außengewinde und dem freien Schaftende statt. Hierbei ist auf dem freien Schaftende der aus fester Spongiosa bestehende Femurkopf durch Zugankerwirkung mindestens einer ihrerseits kopfseitig in der Schenkelhalsplatte verankerten Spongiosa-Schraube fixiert. Nach Art ihrer statischen Funktion können die erfindungsgemäßen Spongiosa-Schrauben deshalb als Zuganker- oder Zuggurtungsschrauben bezeichnet werden.

Die Anzahl der Aufnahmeöffnungen im Fortsatz zur Aufnahme der Spongiosa-Schrauben kann an sich beliebig sein, findet aber regelmäßig ihre praktische Grenze bei der Anzahl vier. Je nach Fraktur-Typ wird es zweckmäßig sein, minimal eine Zuggurtungsschraube oder maximal drei Zuggurtungsschrauben anzuwenden. Irgendeine der vier Aufnahmeöffnungen würde demnach eine von Fall zu Fall einsetzbare Reserve darstellen.

Zusammengefaßt ergeben sich mit dem erfindungsgemäßen Schenkelhalsimplantat folgende Vorteile:

Die Operationstechnik wird erheblich vereinfacht. Es ist kein aufwendiges Zusatzinstrumentarium erforderlich. Die Operationszeit ist wesentlich kürzer. Das Operationstrauma (Weichteilzerstörung, Muskeldurchtrennung etc.) ist erheblich geringer. In der Regel sind auch bei großen Trümmerzonen keine Zusatzosteosynthesen erforderlich. Bei alledem gewährleistet das erfindungsgemäße Schenkelhalsimplantat eine vorteilhafte primäre postoperative Belastung, d.h. eine Belastung - und damit eine frühestmögliche Mobilisierung auch des älteren Patienten - unmittelbar nach der Operation. Bei unvorhersehbaren übergroßen Setzungen des Kopf-Hals-Fragmentes läßt es die Erfindung zu, den Schaft ohne Entfernung des übrigen Implantats in einem vergleichsweise kleinen Eingriff gegen einen axial kürzeren Schaft auszutauschen.

Weitere Vorteile und Erfindungsmerkmale ergeben sich aus den Unteransprüchen sowie aus den Zeichnungen im Zusammenhang mit der Zeichnungsbeschreibung.

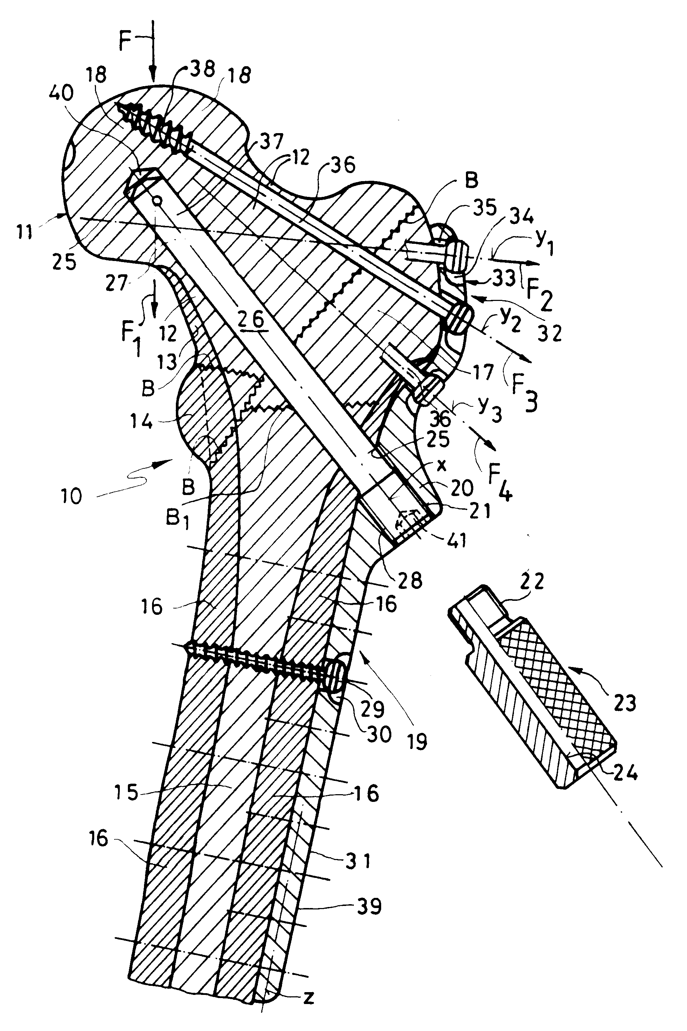

In der einzigen Zeichnung ist die Erfindung anhand eines bevorzugten Ausführungsbeipiels näher dargestellt.

In der Zeichnung ist der obere Bereich eines insgesamt mit 10 bezeichneten Femur im axialen Längsschnitt dargestellt. Das Femur 10 weist folgende hauptsächliche Bereiche auf: den einen Gelenkkopf darstellenden Femurkopf 11, den Schenkelhals 12, den Adam'schen Bogen 13, den Trochanter minor 14, den Markraum 15, die Kortikalis 16 und den Trochanter major 17. Der Tochanter major 17 besteht aus einer feinen wabenartigen Knochensubstanz, der Spongiosa, im Unterschied zur röhrenartigen Kortikalis, die eine kompakte Knochensubstanz darstellt. Besonders dicht - und entsprechend fest - ist die Spongiosa im oberen Bereich 18 des Femurkopfes 11.

Aus der Zeichnung sind gezackte Linien ersichtlich, welche durchgehende Bruchflächen B, B₁ darstellen. Im vorliegenden Fall ist demnach der gesamte Femurkopf 11 einschließlich des Schenkelhalses 12 und ein erheblicher Bereich des Trochanter major 17 abgerissen, wobei erschwerend hinzukommt, daß sich der gesamte Trochanter minor 14 abgelöst hat, der in der Regel noch in sich frakturiert ist. Da die pertrochantäre Bruchzone häufig einen mehrfach frakturierten Trümmerbereich darstellt, fehlt jegliche knöcherne Restabstützung. Es gilt, die Knochenfragmente in ihrer richtigen Lage und so lange Zeit zu positionieren, bis der Knochenbruch geheilt ist und das Femur seine ursprüngliche Festigkeit wiedererlangt hat. Folgende Maßnahmen werden durchgefuhrt:

Die Knochenfragmente werden chirurgisch gerichtet, es wird eine insgesamt mit 19 bezeichnete Schenkelhalsplatte außen an das Femur 10 angesetzt und die Gesamtanordnung gerichteter Femur/Schenkelhalsplatte 19 mit einem geeigneten Hilfsmittel, beispielsweise mit einer Verbrügge-Zange, in der paßgerechten Lage gehalten.

Im oberen Bereich 20 der Schenkelhalsplatte 19, der eine Materialverdickung darstellt, ist ein Innengewinde 21 vorgesehen. In dieses Innengewinde 21 wird der Außengewindeansatz 22 einer Bohrer-Führungshülse 23 eingeschraubt. Die Bohrer-Führungshülse 23 enthält eine zentrisch und axial angeordnete durchgehende Leitbohrung 24, die einen Knochenbohrer mit 6 mm Schaftdurchmesser mit relativ geringem Lagerspiel führen kann. Mit einem nicht dargestellten Pilotbohrer wird sodann eine Pilotbohrung tangential am Adam'schen Bogen vorbei bis in den Femurkopf 11 hinein eingebracht. Sobald die Pilotbohrung erstellt ist, kann deren genaue Lage mittels eines Röntgen-Bildes kontrolliert werden. Bei korrekter Lage der Pilotbohrung wird diese stufenweise bis auf einen Bohrlochdurchmesser von 6 mm aufgebohrt. Anschließend wird bei korrekter Lage der Vorbohrung die Bohrer-Führungshülse 23 herausgeschraubt und durch das Innengewinde 21 hindurch ein Knochenbohrer mit dem endgültigen Kaliber (im vorliegenden Fall: 9 mm) eingeführt und die Vorbohrung auf gesamter axialer Länge erweitert. Der 9 mm-Knochenbohrer führt sich hierbei innerhalb der Vorbohrung von selbst. Es wird so der Aufnahmekanal 25 mit durchgehend glattem kreiszylindrischen Querschnitt erzeugt, in welchen sodann der Schaft 26 eingeführt wird. Der Schaft 26 besteht aus einem massiven durchgehend kreiszylindrischen und außen glatten Zylinderkörper 27 und einem endseitig angeformten Außengewindeansatz 28, welcher (wie in der Zeichnung dargestellt) fest mit dem Innengewinde 21 der Schenkelhalsplatte 19 verschraubt ist. Die Längsmittelachse von Aufnahmekanal 25 und Schaft 26 ist mit x bezeichnet.

Nach Einsetzen des Schaftes 26 und Anpressen der Schenkelhalsplatte 19 an das Femur werden die Kortikalis-Schrauben 29, von denen stellvertretend für eine Vielzahl nur eine einzige dargestellt ist, durch die jeweilige schenkelshalsseitige Aufnahmeöffnung 30 und durch beide Bereiche der Kortikalis 16 hindurch eingeschraubt.

Der unterhalb des verdickten oberen Bereichs 20 angeordnete Längenbereich der Schenkelhalsplatte 19, welcher die Kortikalis-Schrauben 30 aufnimmt, soll als distaler Plattenbereich 31 bezeichnet werden. Die Längsachse des distalen Plattenbereichs 31 ist strichpunktiert angedeutet und mit z bezeichnet. Die Längsachse z bildet mit der Mittellängsachse x einen stumpfen Winkel. In Anpassung an die jeweils vorgegebene individuelle Anatomie kann dieser stumpfe Winkel zwischen 130 und 100° gewählt und bei der Herstellung entsprechend typisiert werden. Auch die Axiallänge des distalen Plattenbereichs ist grundsätzlich variabel, um auch weit nach subtrochantär reichende Frakturen in die Osteosynthese einbeziehen zu können.

Der sich tangential entlang des Adam'schen Bogens 13 erstreckende Schaft 26 befindet sich unterhalb des Trochanter major 17. Ein axialer Überstand der Bohrung 25 vor dem freien Ende des Schafts 26 ist mit 40 bezeichnet.

Oberhalb des verdickten oberen Endbereichs 20 der Schenkelhalsplatte 19 schließt sich ein insgesamt mit 32 bezeichneter Fortsatz an, dessen formlicher Verlauf etwa der Außenkontur des Trochanter major 17 angepaßt ist.

Der Fortsatz 32 weist bis zu vier Aufnahmeöffnungen 33 auf, von denen jede eine konkave Schraubkopfaufnahme 34, und, von dieser ausge-hend, eine durchgehende kreiszylindrische Zen-trieraufnahme 35 besitzt.

Die Aufnahmeöffnungen 33 können auch ohne Zentrieraufnahme 35 ausgebildet sein, so daß eine in Grenzen raumbewegliche Ausrichtung eines Bohrers möglich ist.

Durch die Aufnahmeöffnungen 33 hindurch werden nun Bohrungen eingebracht, die den Trochanter major 17 durchsetzen und bis in die relativ feste Spongiosa des Femurkopfes 11 reichen. In diese Bohrungen werden Spongiosa-Schrauben 36 eingesetzt, von denen in der Zeichnung nur eine vollständig dargestellt ist. Von den anderen teilweise nicht dargestellten Spongiosa-Schrauben 36 sind deren Längsmittelachse und deren Kopfbereich in der Zeichnung eingetragen. Die Längsmittelachsen der Spongiosa-Schrauben 36 sind mit $y_1$, $y_2$ und $y_3$ bezeichnet. Die Spongiosa-Schraube entsprechend der Längsmit-telachse $y_3$ verläuft benachbart dem Schaft 26 und sollte, wenn eben möglich, abweichend von der Zeichnung im wesentlichen parallel zur Längsachse x des Schafts 26 angeordnet sein.

Die Spongiosa-Schraube 36 mit der Längsmit-telachse $y_3$ zieht die Knochenfragmente aneinander und sorgt so für eine enge Anlage im Bereich der Bruchzonen. Der zuvor herausgelöste Trochanter minor 14 wird allerdings mit einer nicht dargestellten Draht-Cerclage gehalten, sofern erforderlich.

Wenn sämtliche Elemente 26, 29, 36 ihre endgültige Lage eingenommen haben, ergeben sich folgende statische Verhältnisse: Die gelenkseitig auf das Femur 10 einwirkende Hüftkraft ist insgesamt mit F bezeichnet. Diese Hüftkraft F wird vektoriell zerlegt, und zwar überträgt der Schaft 26 den Kraftanteil $F_1$, welcher wesentlich geringer ist als die Hüftkraft F. Die erhebliche Differenz zwi-schen F und $F_1$ wird durch Zugkräfte $F_2$, $F_3$ und $F_4$ übernommen, die entlang den Mittellängsachsen $y_1$, $y_2$ und $y_3$ in den als Zuganker bzw. als Zuggurtungsschrauben anzusprechenden Spongiosa-Schrauben 36 wirken. Wichtig ist also, daß die Hüftkraft F nur mit einem wesentlich geringeren Anteil als die Kraft $F_1$ vom Schaft 26 unter Vermei-dung einer schädlichen Biegebelastung aufgenom-men werden muß. Die restlichen Kraftanteile hingegen werden von den Zuggurtungsschrauben 36 aufgenommen und in die insgesamt formsteife Schenkelhalsplatte 19 abgeleitet. Femurseitig erfolgt hierbei der Kraftschluß zwischen dem freien Endbereich 37 des Schaftes 26 und den freien Endbereichen 38 der Spongiosa-Schrauben 36 über die relativ feste, dichte Spongiosa im oberen Bereich des Femurkopfes 11.

Die vorbezeichnete Kraftaufnahme wird noch dadurch verbessert, daß die Mittellängsachsen $y_1$, $y_2$ und $y_3$, und somit die drei Spongiosa-Schrauben 36, fächerförmig angeordnet sind. Insbesondere durch diese Anordnung ergibt sich auch eine große Torsionsfestigkeit der dargestellten Osteo-synthese.

Die Außenseite der Schenkelhalsplatte 19 ist mit 39 bezeichnet. Zur Schraubbetätigung des Schaftes 26 ist ein Innensechskant 41 vorgesehen.

Alle Implantat-Bauteile bestehen aus Implantat-Stahl, aus Titan oder aus einem anderen Metall-oder Nichtmetall-Werkstoff.

## Patentansprüche

1. Schenkelhalsimplantat mit einer länglichen Schenkelhalsplatte (19) welche zu ihrer Befestigung außen am Femur entlang ihrer Längsachse mehrere Aufnahmeöffnungen für Kortikalis-Schrauben (29) aufweist und die an ihrem oberen Bereich mit einem sich stumpfwinklig zu ihrer Längsachse erstreckenden, subtrochantär bis in den Schenkelhals und gegebenenfalls in den Femurkopf hineinreichend zu implantierenden Schaft (26) versehen ist, dadurch gekennzeichnet, daß an den oberen Bereich (20) der länglichen Schenkelhalsplatte (19) ein etwa der Außenkontur des Trochanter major (17) angepaßter bzw. anpaßbarer Fortsatz (32) einstückig angeschlossen ist, der eine oder mehrere Aufnahmeöffnungen (33) für sich pertrochantär bis in den Schenkelhals (12) bzw. bis in den Femurkopf (11) hineinreichend zu befestigende Spongiosa-Schrauben (36) aufweist, und daß der Schaft (26) durchgehend außen glatt und kreiszylindrisch ausgebildet und mit einem endseitig angeformten Außengewindeansatz (28) versehen ist, mittels welchem er in einer Innengewindeaufnahme (21) im oberen Bereich (20) der länglichen Schenkelhalsplatte (19) lösbar befestigt ist.

2.  Schenkelhalsimplantat nach Anspruch 1, dadurch gekennzeichnet, daß die Schenkelhalsplatte (19) im Bereich der Innengewindeaufnahme (21) verstärkt ist.

3.  Schenkelhalsimplantat nach Anspruch 1 oder nach Anspruch 2, dadurch gekennzeichnet, daß eine Bohrer-Führungshülse (23) mit einer zentrisch und axial verlaufenden durchgehenden Leitbohrung (24) zur Führung eines Knochenbohrers einen in die schenkelhalsplattenseitige Innengewindeaufnahme (21) einsetzbaren Außengewindeansatz (22) aufweist.

4.  Schenkelhalsimplantat nach einem der Ansprüche 1 bis 3, dadurch gekennzeichnet, daß die Aufnahmeöffnungen (33) für die Spongiosa-Schrauben (36) je eine von der Außenseite (39) der Schenkelhalsplatte (19) weg weisende kreiszylindrische Zentrieraufnahme (35) zur Axialführung eines Bohres bzw. des Schaftes der Spongiosa-Schraube (36) aufweisen.

5.  Schenkelhalsimplantat nach Anspruch 4, dadurch gekennzeichnet, daß die Längsachse (bei $y_3$) der Zentrieraufnahme (35) für die dem Schaft unmittelbar benachbarte Spongiosa-Schraube (36) im wesentlichen parallel zur Längsachse (x) des Schaftes (26) verläuft.

6.  Schenkelhalsimplantat nach einem der Ansprüche 1 bis 5, dadurch gekennzeichnet, daß die Aufnahmeöffnungen (33) bzw. die Zentrieraufnahmen (35) für die Spongiosaschrauben (36) deren fächerförmige Anordnung zueinander gestatten oder vorgeben.

**Claims**

1.  Femoral neck implant having an oblong femoral neck plate (19), which for its fastening externally on the femur has a plurality of location openings for cortical screws (29) along its longitudinal axis and which is provided at its upper region with a shaft (26) which extends at an obtuse angle to the longitudinal axis of said plate and is to be implanted so as to reach subtrochanterically as far as into the neck and possibly into the head of the femur, characterized in that there is integrally connected to the upper region (20) of the oblong femoral neck plate (19) an extension (32), which is adapted or is adaptable, for example, to the external contour of the greater trochanter (17) and has one or more location openings (33) for spongiosa screws (36) to be fastened so that they extend pertrochanterically as far as into the neck (12) or the head (11) of the femur, and that the shaft (26) is of a continuously externally smooth and circular cylindrical construction and has formed on one end an externally threaded attachment (28), by means of which it may be detachably fastened in an internally threaded seat (21) in the upper region (20) of the oblong femoral neck plate (19).

2.  Femoral neck implant according to claim 1, characterized in that the femoral neck plate (19) is strengthened in the region of the internally threaded seat (21).

3.  Femoral neck implant according to claim 1 or according to claim 2, characterized in that a drill finder sleeve (23) with a centrically and axially extending continuous guide bore (24) has, for guiding a bone drill, an externally threaded attachment (22) which may be inserted into the internally threaded seat (21) on the side of the femoral neck plate.

4.  Femoral neck implant according to one of claims 1 to 5, characterized in that the location openings (33) for the spongiosa screws (36) each have a circular cylindrical centring seat (35) directed away from the exterior (39) of the femoral neck plate (19) for axially guiding a drill or the shaft of the spongiosa screw (36).

5.  Femoral neck implant according to claim 4, characterized in that the longitudinal axis (at $y_3$) of the centring seat (35) for the spongiosa screw (36) directly adjacent to the shaft extends substantially parallel to the longitudinal axis (x) of the shaft (26).

6.  Femoral neck implant according to one of claims 1 to 5, characterized in that the location openings (33) and the centring seats (35) for the spongiosa screws (36) allow or dictate the arrangement of said screws in a fanned-out manner relative to one another.

**Revendications**

1.  Implant de col de fémur comprenant une plaque de col de fémur (19) de forme allongée, qui présente, pour sa fixation contre la face extérieure du fémur, plusieurs ouvertures de logement réparties le long de son axe longitudinal, pour recevoir des vis à corticale (29), et qui est muni, dans sa région supérieure, d'une broche (26) s'étendant dans une direction qui forme un angle obtus avec l'axe longitudinal de la plaque, et qui doit être implantée dans une position sous-trochantérienne, jusque dans le col du fémur et éventuellement prolongée jusque dans la tête du fémur,
caractérisé en ce qu'a' la région supérieure (20) de la plaque allongée (19) de col de fémur, se raccorde un prolongement (32) approximativement adapté ou adaptable au profil extérieur du grand trochanter (17), d'une seule pièce avec cette région, qui présente une ou plusieurs ouvertures de logement (33) destinées à recevoir des vis (36) à spongieuse qui doivent être fixées dans une position pertrochantérienne, en se prolongeant jusque dans le col (12) du fémur ou jusque dans la tête (11) du fémur, et en ce que la broche (26) est d'une configuration extérieurement lisse et cylindrique à base circulaire sur toute sa longueur, et est munie d'un embout (28) fileté extérieurement, formé à son extrémité, au moyen duquel elle est fixée de façon démontable dans un logement (21) fileté intérieurement prévu dans la région supérieure (20) de la plaque de col de fémur (19) de forme allongée.

2. Implant de col de fémur selon la revendication 1,
caractérisé en ce que la plaque de col de fémur (19) est renforcée dans la région du logement (21) à filetage intérieur.

3. Implant de col de fémur selon la revendication 1 ou la revendication 2,
caractérisé en ce qu'un canon de guidage d'alésoir (23), possédant un alésage de guidage (24) qui le traverse entièrement en position centrale et en direction axiale, destiné au guidage d'un alésoir, présente un embout (22) à filetage extérieur qui se visse dans le logement à filetage intérieur (21) prévu dans la plaque du col du fémur.

4. Implant de col de fémur selon une des revendications 1 à 3,
caractérisé en ce que les ouvertures (33) destinées à recevoir les vis à spongieuse (36) présentent chacune un logement de centrage (35) cylindrique à base circulaire, qui part de la face extérieure (39) de la plaque de col de fémur (19) et qui sert au guidage axial d'un foret ou de la tige de la vis à spongieuse (36).

5. Implant de col de fémur selon la revendication 4,
caractérisé en ce que l'axe longitudinal (en $y_3$) du logement de centrage (35) destiné à recevoir la vis à spongieuse (36) qui est immédiatement adjacente à la broche s'étend sensiblement parallèlement à l'axe longitudinal (x) de la broche (26).

6. Implant de col de fémur selon une des revendications 1 à 5,
caractérisé en ce que les ouvertures de logement (33) ou les logements de centrage (35) destinés à recevoir les vis à spongieuse (36) permettent ou imposent une disposition en éventail de ces vis les unes par rapport aux autres.